# EUROPEAN PATENT APPLICATION

(11) **EP 2 196 197 A1**
(43) Date of publication of application: **16.06.2010**
(21) Application number: 08171696.1
(22) Date of filing: 15.12.2008
(51) Int. Cl.: A61K 9/70, A61K 31/52, A61K 31/522, A61K 38/00, A61K 38/21

(54) **Antiviral patch**

(71) Applicant: Bouty S.P.A., 20129 Milano (IT)
(72) Inventor: Marra, Fabio, 20129, MILANO (IT); Di Grigoli, Maurizio, 20129, MILANO (IT); Comuzio, Sergio, 20129, MILANO (IT)
(74) Representative: Minoja, Fabrizio

(57) **Abstract**

A patch comprising a skin adhesive layer, a backing layer and a release liner, the adhesive layer comprising an antiviral agent, a low volatile solvent, and a polymeric adhesive soluble in highly volatile solvents.

## Description

The invention refers to a patch comprising a skin adhesive layer, a backing layer and a release liner, the adhesive layer comprising an antiviral agent, a low volatile solvent, and a polymeric adhesive soluble in highly volatile solvents.

### Background of the invention

Herpes simplex labialis (HSL) is characterized by recurrent cold sores resulting primarily from herpes simplex virus type 1 infection of the lips and perioral skin. HSL is prevalent, occurring in approximately 15% to 40% of adults worldwide (1-4).

Topical antiviral therapies, such as acyclovir and penciclovir cream or ointment, are commonly used for treating subjects with HSL (5,6), but overall benefits of these therapies may be limited by several factors, including the need for frequent application, lack of protection of the lesion, formation of a scab and aesthetic factors relating to the visibility of the drug product on the site of application or appearance of the lesion during the healing process.

Many of the limitations of standard topical antiviral therapy for the treatment of HSL lesions may be addressed by appropriate wound management. Recent clinical evidence has suggested that HSL lesions are deeper than epidermal wounds and may be classified as partial-thickness wounds that extend through the basal membrane into the dermis (7).

Protection of HSL lesions from foods or fluids, general mouth activity and external environmental factors may be critical in reducing pain and promoting wound re-epithelization and proper healing.

According to a recent study, 61% of 998 health care professionals, who treat recurrent HSL, reported that the social embarrassment of having a cold sore was a major concern (8).

Likewise, a recent comprehensive review of psycho-dermatologic disorders listed herpes simplex virus infection as a disorder associated with a variety of psychopathologic problems that may be exacerbated by emotional stress (9).

One or more drug treatments for skin administration, including acyclovir cream and ointment, penciclovir cream and idoxuridine in dimethyl sulfoxide (DMSO) (10), are commercially available, but their administration is inconvenient, needing frequent applications for several days, especially in the early stage of the development of the pathology. Moreover the visibility of the product can reduce the patients' compliance.

On the other side, a number of treatments are available over the counter, comprising either active ingredients reducing lesion pain or promoting wound healing through occlusion, without any anti-viral agent, such as for instance the product Compeed^{®} marketed by Johnson & Johnson (11).

### Description of the invention

The present invention provides a device to treat and simultaneously to cover and protect the herpes viral lesions. For these purposes, the present invention provides a water insoluble self-adhesive patch useful to deliver continuously anti-viral drugs to the sites of the body that could be normally infected by Herpes Simplex or by Herpes Zoster Viruses.

The use of the patch of the invention is very practical for the patient. The patch is effective in delivering drugs to the skin and compared to the conventional creams or ointments, it provides a continuous drug delivery without repeated daily applications. Moreover, it is a cosmetic aid which helps the patient also from the psychological point of view, masking cold sores.

More particularly, the patch of the invention comprises a skin adhesive layer, a backing layer and a release liner, the adhesive layer comprising an antiviral agent, a low volatile solvent and a polymeric adhesive soluble in highly volatile solvents.

The antiviral agent is selected from acyclovir, penciclovir, famciclovir, idoxuridine, interferon, edoxudine, preferably acyclovir, pencivlovir or famciclovir.

Said antiviral agent may be included into a separate reservoir layer, or it can be incorporated in the adhesive layer, in an amount from 0.1 to 10% by weight, dissolved in a low volatile solvent.

The low volatile solvent has typically an high boiling point (in the range of 150°C-250°C), and low vapor pressure. Preferred low volatile solvents are selected from dimethylacetamide, dimethylformamide, dimethylsulfoxide, ethylene glycol, polyethylene glycol, xylene, propylene glycol, pyrrolidone, glycerol, 1,4-dioxane, triethanolamine or mixtures thereof. Dimethylsulfoxide is particularly preferred.

The low volatile solvent is preferably present in an amount from 10 to 50% by weight.

The adhesive polymer is selected from pectin, agar gum, acacia gum, cellulose, cellulose derivatives, xanthan gum, polyvinyl alcohol, polymethacrylic acid, polymethacrylate, acrylates/alkylmethacrylates copolymers, polyvinyl pyrrolidone, polyvinyl alcohol, cellulose or cellulose derivatives, such as hydroxypropylcellulose, hydroxyethylcellulose, or blends thereof.

The adhesive layer is formed from a solution of the adhesive polymer in an highly volatile solvent, i.e having a low boiling point (in the range of 40°C-100°C) and high vapor pressure. Said solvent is then usually evaporated during the manufacturing process even though a certain amount, up to 15% by weight, may be left in the adhesive layer after drying.

The adhesive polymer or the adhesive polymer blend is present in an amount from 20 to 50% by weight.

The patch may further contain as a non polymeric crystallization inhibitors citric acid, succinic acid, lactic acid, and esters thereof, in an amount from 0.5 to 15% by the weight.

The patch may also contain other excipients such as cross-linkers, penetration enhancers, plasticizers, fragrances, emollients.

The backing layer is transparent, semi-occlusive or occlusive, oxygen permeable, preferably consisting of polyurethane film, polyethylene, ethylene vinyl acetate or polyolephine film with a MVTR (moisture vapor transmission rate) from 50 to 3500 g/m²/day and a thickness from 40 to 150 µm.

The backing layer is very flexible and soft, transparent or colored and can be occlusive or perspirating, providing a masking effect of the cold sore. Moreover, it protects the damaged skin and the viral lesions from the external contact, thus reducing the patient's pain and the possibility of further contaminations or infections, and improving the re-epithelization process.

The adhesive layer is protected from the external environment through a release liner, that has to be removed before applying the patch to the site of the body interested by the viral lesions. Once the patch is applied, through the self-adhesive layer, it can be kept on-site up to 12 hours, delivering the active ingredient into and across the skin.

The patches of the invention are prepared by a process comprising the steps of blending the solution of adhesive polymers in highly volatile solvents together with the other components and then casting the mixture on a silicone coated liner film, before drying and the final lamination.

The highly volatile solvents evaporate, leaving the adhesive film on the release liner whereas the low volatile solvent remains in the dry adhesive layer preventing the drug crystallization. Optionally, if the drug is present in a reservoir layer, the amount of low volatile solvent can be higher.

The polymers used according to this invention are those normally used to produce pressure sensitive adhesives (PSAs) or bio-adhesive film in an organic or aqueous solution, in a concentration ranging from 20% to 80%, preferably from 20 to 50% of the composition of the adhesive, while the concentrations of the highly volatile solvent are from 10% to 50%. Other components of the adhesive layer or of the reservoir layer include thickening agents, chemical permeation enhancers, non-polymeric crystallization inhibitors, flavours, surfactants, cross-linkers, buffering agents, plasticizers, preservatives, anti-oxidants, pigments.

The selected solvents and polymers must of course be compatible and form an homogeneous solution which may be uniformly casted.

Low boiling point solvents, i.e. high volatile solvents, are preferably water, ethanol, methanol, isopropyl alcohol, ethyl acetate, more preferably water.

This invention actually allows to produce an anti-herpes patch, having an effective amount of anti-viral drug that can be delivered to the site of application.

The low volatile solvent helps to avoid crystallization by keeping dissolved the active substances in the matrix and affects the diffusivity of the drug through the matrix, to reach the skin and the site of action. The matrix must be chosen according to the physical-chemical properties of the low volatile solvents or the solvents blend. The polymer must provide a good cohesiveness to the final product. The quantitative composition of the adhesive blend is chosen in order to have an acceptable film in terms of thickness, cohesion properties, mechanical resistance, skin adhesion, peel properties and handling.

The polymer blend range in the dry matrix is 5%-50%, most preferably 20%-35%, solubilized in a low boiling point solvent or solvent mixture. The solvent percentage range is from 20% to 70%, preferably from 35% to 55%, in the mixture that has to be casted to produce the adhesive layer or the reservoir layer. In the dry matrix the amount of low boiling point solvent must not exceed 15% by weight.

The low volatile solvents, instead, are included in the dry matrix, entangled in the polymer and dissolving the active ingredients. The amount of these solvents in the dry state is in the range 10%-50%, but preferably in the range 30%-55%.

The invention is described in more detail in the following examples.

### Example 1 - ACV019FM composition

A patch formulation is disclosed comprising 3.7% of acyclovir in the dry matrix and as adhesive a blend of PVP (Kollidon^{®} K 90 P) and a water solution of acrylates/dimethylamino methacrylates copolymer, known as Plastoid^{®}. Table 1 reports the composition of the formulation. Acyclovir is solubilized in dimethyl sulfoxide, known also to improve permeation of active substances through the skin.

The transparent solution is mixed to the adhesive blend, and, once homogeneity is reached, the mass is film-casted on the release liner and dried in oven.

The patch is completed by laminating the backing layer on the adhesive layer and cutting in the opportune dimension and shape.

**Table 1: ACV019FM composition**

| **Ingredients** | **% wet matrix** | **% dry matrix** |
|---|---|---|
| Water (volatile solvent) | **40.5** | **-** |
| Water from Plastoid^{®} (volatile solvent) | **12.0** | **-** |
| PEG 400 (plasticizer) | **7.4** | **14.1** |
| DMSO (low volatile solvent) | **22.7** | **43.5** |
| PVP K 90 P (adhesive polymer component) | **13.1** | **25.0** |
| Citric acid (crystallization inhibitor) | **2.5** | **4.8** |
| Acyclovir | **1.9** | **3.7** |
| Plastoid solid (adhesive polymer blend) | **4.6** | **8.9** |
| TOT | 100.00 | 100.0 |

### Example 2 - PEN001FM

A patch containing penciclovir is described. The preparation steps are similar to the previous. Table 2 reports the composition of the formulation.

**Table 2: PEN001FM composition**

| **Ingredients** | **% wet matrix** | **% dry matrix** |
|---|---|---|
| Water (volatile solvent) | **45.4** | **-** |
| PEG 400 (plasticizer) | **6.8** | **11.9** |
| DMSO (low volatile solvent) | **26.6** | **46.6** |
| PVP K 90 P (adhesive polymer) | **14.2** | **25.0** |
| Diethyl citrate (crystallization inhibitor) | **6.2** | **11.0** |
| Penciclovir | **0.3** | **1.4** |
| Polyvinyl alcohol (adhesive polymer blend) | **2.3** | **4.1** |
| TOT | 100.00 | 100.0 |

### Example 3 - Transepidermal permeation of acyclovir through separated epidermis of pig ear skin

In vitro permeation of the acyclovir formulations was performed over epidermis obtained from porcine inner ear skin, using Franz diffusion cells and a receptor solution of degassed phosphate-buffered saline solution (PBS). A 5% acyclovir commercial cream was employed as reference (Cycloviran®). Drug permeation rate through the epidermis and the amount of acyclovir accumulated at the end of the experiments were evaluated by HPLC analysis.

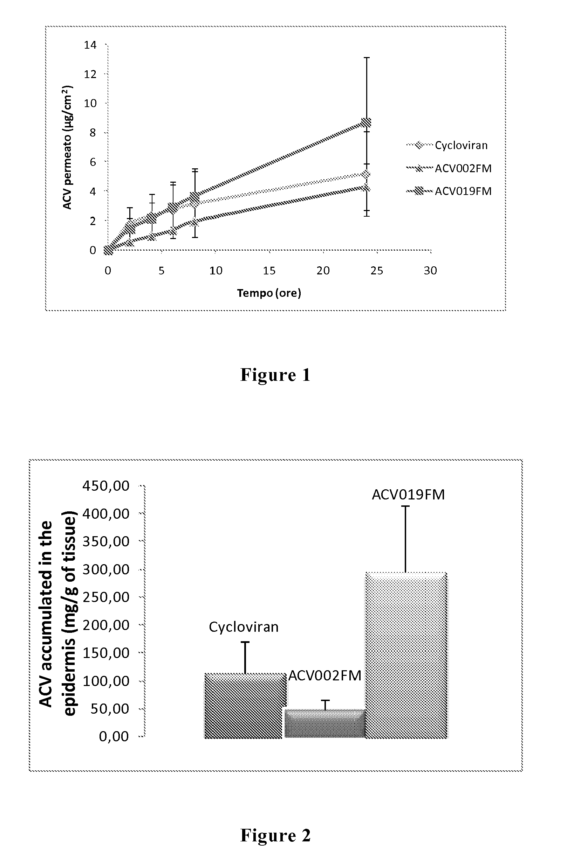

Figure 1 describes the permeation profile of acyclovir through porcine epidermis, comparing the drug permeated from two patches with the amount permeated from the commercial reference.

Acyclovir permeation rate across the epidermis from the formulation ACV002FM is not significantly different from the amount of drug permeated from the commercial reference formulation, while the permeation profile of acyclovir generated following the application of ACV019FM is significantly higher, being the patch more effective in acyclovir delivery across the skin.

In figure 2 the amount of acyclovir recovered in the skin after the permeation experiment is reported.

Also in this data analysis it is possible to notice that the amount of drug recovered in the epidermis after 24 hours of application of ACV002FM is not statistically significantly different from Cycloviran®. On the other side ACV019FM provided higher amount of ACV in the epidermis than the reference.

### References

1) Embil JA, Can Med Assoc J 1975; 113: 627-630.
2) Lowhagen GB, Scand J Infect Dis 2002; 34: 664-667.
3) Malvy D, et al. J Eur Acad Dermatol Venereol 2007; 21: 1398-1403.
4) Young TB, Am J Epidemiol 1988; 127: 612-625.
5) Spruance SL, Antimicrob Agents Chemother 2002; 46: 2238-2243.
6) Raborn GW, et al.. J Am Dent Assoc 2002; 133: 303-309.
7) Patel AR, Adv Skin Wound Care 2007; 20: 408-412.
8) Raborn GW, J Am Dent Assoc 2004; 135: 48-54.
9) Jafferany M. Prim Care Companion J Clin Psychiatry 2007; 9: 203-213.
10) Hamuy R, Eur J Derm, 1998; 8: 310-319.
11) Karlsmark T et al, JEADV 2008: 1-9.

## Claims

1. A patch comprising a skin adhesive layer, a backing layer and a release liner, the adhesive layer comprising an antiviral agent, a low volatile solvent, and a polymeric adhesive soluble in highly volatile solvents.

2. The patch of claim 1, wherein the active agent is selected from acyclovir, penciclovir, famciclovir, idoxuridine, interferon, edoxudine.

3. The patch of claim 1 or 2, wherein the content of an antiviral agent is from 0.1 to 10% by weight.

4. The patch of any one of claims 1-3, wherein the low volatile solvent is selected from dimethyl sulfoxide, propylene glycol, glycerol, xylene, dimethyl acetamide, 1,4-dioxane, triethanolamine or mixtures thereof.

5. The patch of any one of claims 1-4 wherein the content of the low volatile solvent is from 10 to 50% by weight.

6. The patch of any one of claims 1-5, wherein the adhesive polymer is selected from pectin, agar gum, acacia gum, cellulose, cellulose derivatives, polyvinyl pyrrolidone xanthan gum, polyvinyl alcohol, polyacrylic acid, methacrylate derivatives or blends thereof.

7. The patch of any one of claims 1-6, wherein the adhesive polymer or the adhesive polymer blend is present in an amount from 20 to 80% by weight.

8. The patch of any one of claims 1-7, further containing a non polymeric crystallization inhibitor selected from citric acid, succinic acid, lactic acid, and esters thereof, in an amount from 0.5 to 15% by the weight.

9. The patch of any one of claims 1-8, further containing other excipients selected from cross-linkers, penetration enhancers, plasticizers, fragrances, emollients.

10. The patch of any one of claims 1-9, wherein the backing layer is transparent, semiocclusive or occlusive, oxygen permeable.

11. The patch according to claim 10 wherein the backing layer is polyurethane film, polyethylene, ethylene vinyl acetate or polyolephine film with a MVTR (moisture vapor transmission rate) from 50 to 3500 g/m²/day and a thickness from 40 to 150 µm.

12. The patch of any one of claims 1-11 wherein the highly volatile solvent is selected from water, ethanol, acetone, isopropanol, acetic acid, isobutanol, ethyl acetate, methyl ethyl ketone.
